(19) **Europäisches Patentamt**
**European Patent Office**
**Office européen des brevets**

(11) Publication number: **0 437 621 A1**

## (12) EUROPEAN PATENT APPLICATION
### published in accordance with Art. 158(3) EPC

(21) Application number: **90910189.1**

(22) Date of filing: **06.07.90**

(86) International application number:
**PCT/JP90/00872**

(87) International publication number:
**WO 91/00738 (24.01.91 91/03)**

(51) Int. Cl.5: **A61K 37/24**

(30) Priority: **07.07.89 JP 176535/89**

(43) Date of publication of application:
**24.07.91 Bulletin 91/30**

(84) Designated Contracting States:
**AT BE CH DE DK ES FR GB IT LI LU NL SE**

(71) Applicant: **KYOWA HAKKO KOGYO CO., LTD.**
**Ohtemachi Bldg., 6-1 Ohtemachi I-chome**
**Chiyoda-ku Tokyo 100(JP)**

(72) Inventor: **UENO, Yasuhiko** 410-1, Nameri
**Nagaizumi-cho**
**Sunto-gun**
**Shizuoka 411(JP)**
Inventor: **NAGANUMA, Hirotake** 198-1,Kakita
**Shimizu-cho**
**Sunto-gun**
**Shizuoka 411(JP)**
Inventor: **TERAJIMA, Mitsuru** 110-1, Honjuku
**Nagaizumi-cho Sunto-gun**
**Shizuoka 411(JP)**
Inventor: **HAYAKAWA, Eiji**
**495-15, Chabatake Susono-shi**
**Shizuoka 410-11(JP)**

(74) Representative: **Vossius & Partner**
**Siebertstrasse 4 P.O. Box 86 07 67**
**W-8000 München 86(DE)**

(54) **AQUEOUS SOLUTION OF MOTILIN.**

(57) An aqueous solution of motilin, having a pH value adjusted to 4.0 to 5.5 by a pharmaceutically acceptable pH regulator, and lyophilized motilin prepared by freeze-drying said solution.

EP 0 437 621 A1

## TECHNICAL FIELD

The present invention relates to a stable aqueous solution containing a motilin and to a freeze-dried preparation thereof. Motilin is a gastrointestinal hormone which stimulates gastrointestinal motility in hunger and is expected to be useful as a drug for, e.g., improving gastrointestinal motility.

Background Art

In recent years, with the development of synthetic technology and recombinant DNA technology, it has become possible to produce many physiologically active proteins and peptides. Motilins in the present invention can be produced by peptide synthesis and recombinant DNA technology. Motilins have poor stability in solution and in a solid state, like general physiologically active peptides. No method is known for stabilizing an aqueous solution or freeze-dried preparation of motilins.

Disclosure of the Invention

The present invention is based on the finding that an aqueous solution of a motilin and its freeze-dried preparation can be improved in stability by adjusting the pH to 4.0 to 5.5 and can be provided for practical use.

The motilins to be used in the present invention are not particularly limited, and usually usable ones such as swine motilin, canine motilin, and human motilin [it has been confirmed that human motilin has the same structure as that of swine motilin, FEBS LETTER, 223, 1, 74 (1987)] can be used. In addition, there may also be used motilin derivatives, e.g., [13]leucine-swine motilin wherein the methionine at the 13-position of swine motilin is substituted with leucine (Japanese Published Unexamined Patent Application No. 71195/88). These motilins may be either in a free state or in the form of an organic or inorganic acid salt.

The pH controllers to be used in the present invention are not particularly limited but are those capable of maintaining the pH at 4.0 to 5.5.

Specific examples of the pH controllers include acetic acid-sodium acetate, potassium hydrogenphthalate-sodium hydroxide, sodium secondary citrate-hydrochloric acid, glycine-sodium chloride-hydrochloric acid, sodium secondary citrate-sodium hydroxide, monopotassium phosphate-disodium phosphate, monosodium phosphate-disodium phosphate, monosodium phosphate-dipotassium phosphate, monopotassium phosphate-dipotassium phosphate, tartaric acid-sodium tartrate, lactic acid-sodium lactate, sodium Veronal-sodium acetate-hydrochloric acid, succinic acid-borax, potassium primary citrate-sodium hydroxide, potassium primary citrate-borax, disodium phosphate-citric acid, sodium acetate-hydrochloric acid, glutamic acid-sodium hydroxide, and aspartic acid-sodium hydroxide.

The concentration of the pH controller is not particularly limited but is preferably in the range of 10 mM to 1 M, more preferably 50 mM to 100 mM, as a concentration in an aqueous solution.

Motilins are stabilized by dissolving them in an aqueous solution adjusted to pH 4.0 to 5.5 by using the pH controller. For example, when [13]leucine-swine motilin was dissolved in an aqueous solution adjusted to pH 4.5 and the solution was stored at $60°C$ for 7 days, the residual motilin content was approximately 94%. On the other hand, the motilin content of an aqueous solution adjusted to pH 3.0 lowered to about 54% after storage at $60°C$ for 7 days, and that of an aqueous solution adjusted to pH 6.0 lowered to about 59% after storage at $60°C$ for 7 days. Similar results were obtained with [13]methionine-swine motilin which is a naturally occurring motilin.

The concentration of a motilin in the aqueous solution is 0.01 $\mu$g to 100 mg/ml, preferably 1 $\mu$g/ml to 1 mg/ml. The freeze-dried preparation of motilin can be obtained by conventional methods. That is, 0.1 to 10 mg/ml of a motilin is dissolved in an aqueous solution adjusted to pH 4.0 to 5.5 and the solution is charged into a suitable container, followed by freeze-drying by using a freeze-drier. For example, an aqueous solution of motilin is charged into a sterile glass vial, frozen at a temperature below $-50°C$ for 4 hours, and then dried at $-10°C$ for 36 hours in vacuum at 0.05 mbar. Then, the vial is subjected to secondary drying at $40°C$ for 8 hours. After completion of the drying, the vial is filled with sterile dry nitrogen gas until the atmospheric pressure is reached. Thereafter, the vial is stoppered with a rubber stopper for freeze-drying and then sealed with an aluminum cap. In this case, an excipient such as mannitol or lactose may be added to improve the appearance of a cake of the freeze-dried preparation.

The aqueous solution and freeze-dried preparation of the present invention may also contain a preservative, a stabilizer, an excipient, a binder, a disintergrator, a wetting agent, a lubricant, a coloring agent, an aromatic, a corrigent, a coating, a suspending agent, an emulsifier, a dissolution aid, a buffer, an isotonic agent, a plasticizer, a surfactant, an analgesic, etc. which are pharmaceutically acceptable.

Example 1

To 10 mg of [13]lencine-swine motilin was added 1/15 M phosphate buffer previously adjusted to pH 4.5 to make the volume 100 ml. Thus, 100 μg/ml aqueous solution of [13]leucine-swine motilin was obtained.

Example 2

To 10 mg of [13]leucine-swine motilin was added 1/15 M phosphate buffer previously adjusted to pH 4.5 to make the volume 100 ml. The obtained 100 μg/ml aqueous solution of [13]leucine-swine motilin was filled into vials in 2 ml portions and freeze-dried in a conventional manner to give a freeze-dried preparation of [13]leucine-swine motilin.

Example 3

To 10 mg of [13]methionine-swine motilin was added 1/15 M phosphate buffer previously adjusted to pH 4.5 to make the volume 100 ml. Thus, 100 μg/ml aqueous solution of [13]methionine-swine motilin was obtained.

Example 4

To 10 mg of [13]methionine-swine motilin was added 1/15 M phosphate buffer previously adjusted to pH 4.5 to make the volume 100 ml. The obtained 100 μg/ml aqueous solution of [13]methionine-swine motilin was filled into vials in 2 ml portions and freeze-dried in a conventional manner to give a freeze-dried preparation of [13]methionine-swine motilin.

Test Example 1

The aqueous solution of [13]leucine-swine motilin obtained in Example 1 was stored at 60°C for 7 days, and the residual motilin content was determined by the HPLC method.

For comparison, [13]leucine-swine motilin was dissolved in phosphate buffer adjusted to pH 3.0 to give 100 μg/ml aqueous solution of [13]leucine-swine motilin (Comparative Example 1).

In a similar manner, [13]leucine-swine motilin was dissolved in phosphate buffer adjusted to pH 6.0 to give 100 μg/ml aqueous solution of [13]leucine-swine motilin (Comparative Example 2).

The samples obtained in Comparative Examples 1 and 2 were stored at 60°C for 7 days, and the residual motilin content was determined by the HPLC method. The results are shown in Table 1.

Table 1

| | pH | Residual Motilin Content (%) after Storage at 60°C for 7 days |
|---|---|---|
| Example 1 | 4.5 | 94.1 |
| Comparative Example 1 | 3.0 | 54.0 |
| Comparative Example 2 | 6.0 | 59.4 |

Test Example 2

The freeze-dried preparation of [13]leucine-swine motilin obtained in Example 2 was stored at 80°C for 7 days, and the residual motilin content was determined by the HPLC method.

For comparison, [13]leucine-swine motilin was dissolved in the buffer adjusted to pH 7.0 and the solution was freeze-dried in a conventional manner (Comparative Example 3). The sample obtained in Comparative Example 3 was stored at 80°C for 7 days, and the residual motilin content was determined by the HPLC method. The results are shown in Table 2.

### Table 2

| | pH | Residual Motilin Content (%) after Storage at 80°C for 7 days |
|---|---|---|
| Example 2 | 4.5 | 62.3 |
| Comparative Example 3 | 7.0 | 32.9 |

Test Example 3

The aqueous solution of [13]methionine-swine motilin obtained in Example 3 was stored at 60°C for 7 days, and the residual [13]methionine-swine motilin content was determined by the HPLC method.

For comparison, [13]methionine-swine motilin was dissolved in phosphate buffer adjusted to pH 3.0 to give 100 $\mu$g/ml aqueous solution of [13]methionine-swine motilin (Comparative Example 4).

In a similar manner, [13]methionine-swine motilin was dissolved in phosphate buffer adjusted to pH 6.0 to give 100 $\mu$g/ml aqueous solution of [13]methionine-swine motilin (Comparative Example 5).

The samples obtained in Comparative Examples 4 and 5 were stored at 60°C for 7 days, and the residual [13]methionine-swine motilin content was determined by the HPLC method. The results are shown in Table 3.

### Table 3

| | pH | Residual Motilin Content (%) after Storage at 60°C for 7 days |
|---|---|---|
| Example 3 | 4.5 | 95.7 |
| Comparative Example 4 | 3.0 | 57.1 |
| Comparative Example 5 | 6.0 | 58.5 |

Test Example 4

The freeze-dried preparation of [13]methionine-swine motilin obtained in Example 4 was stored at 80°C for 7 days, and the residual [13]methionine-swine motilin content was determined by the HPLC method.

For comparison, [13]methionine-swine motilin was dissolved in the buffer adjusted to pH 7.0 and the solution was freeze-dried in a conventional manner (Comparative Example 6). The sample obtained in Comparative Example 6 was stored at 80°C for 7 days, and the residual [13]methionine-swine motilin content was determined by the HPLC method. The results are shown in Table 4.

EP 0 437 621 A1

Table 4

|  | pH | Residual Motilin Content (%) after Storage at 80°C for 7 days |
|---|---|---|
| Example 4 | 4.5 | 71.5 |
| Comparative Example 6 | 7.0 | 35.9 |

**Claims**

1. An aqueous solution comprising a motilin and adjusted to pH 4.0 to 5.5 by using a pharmaceutically acceptable pH controller.

2. A freeze-dried preparation of a motilin which is obtained by freeze-drying an aqueous solution comprising the motilin and adjusted to pH 4.0 to 5.5 by using a pharmaceutically acceptable pH controller.

# INTERNATIONAL SEARCH REPORT

International Application No  PCT/JP90/00872

## I. CLASSIFICATION OF SUBJECT MATTER (if several classification symbols apply, indicate all) [6]

According to International Patent Classification (IPC) or to both National Classification and IPC

Int. Cl[5]     A61K37/24

## II. FIELDS SEARCHED

### Minimum Documentation Searched [7]

| Classification System | Classification Symbols |
|---|---|
| IPC | A61K37/24 |

### Documentation Searched other than Minimum Documentation to the Extent that such Documents are Included in the Fields Searched [8]

## III. DOCUMENTS CONSIDERED TO BE RELEVANT [9]

| Category [*] | Citation of Document, [11] with indication, where appropriate, of the relevant passages [12] | Relevant to Claim No. [13] |
|---|---|---|
| P | EP, A, 369437 (Kyowa Hakko Kogyo Co., Ltd.), 23 May 1990 (23. 05. 90), Claim (Family: none) | 1 - 2 |
| A | JP, A, 62-26234 (The Wellcome Foundation Ltd.), 4 February 1987 (04. 02. 87), Claim & GB, A, 2176703 | 1 - 2 |

* Special categories of cited documents: [10]

"A"  document defining the general state of the art which is not considered to be of particular relevance

"E"  earlier document but published on or after the international filing date

"L"  document which may throw doubts on priority claim(s) or which is cited to establish the publication date of another citation or other special reason (as specified)

"O"  document referring to an oral disclosure, use, exhibition or other means

"P"  document published prior to the international filing date but later than the priority date claimed

"T"  later document published after the international filing date or priority date and not in conflict with the application but cited to understand the principle or theory underlying the invention

"X"  document of particular relevance; the claimed invention cannot be considered novel or cannot be considered to involve an inventive step

"Y"  document of particular relevance; the claimed invention cannot be considered to involve an inventive step when the document is combined with one or more other such documents, such combination being obvious to a person skilled in the art

"&"  document member of the same patent family

## IV. CERTIFICATION

| Date of the Actual Completion of the International Search | Date of Mailing of this International Search Report |
|---|---|
| September 11, 1990 (11. 09. 90) | October 1, 1990 (01. 10. 90) |

| International Searching Authority | Signature of Authorized Officer |
|---|---|
| Japanese Patent Office | |

Form PCT/ISA/210 (second sheet) (January 1985)